# EUROPEAN PATENT SPECIFICATION

(11) **EP 3 603 594 B1**
(45) Date of publication and mention of the grant of the patent: **24.02.2021**
(21) Application number: 18774839.7
(22) Date of filing: 16.03.2018
(51) Int. Cl.: A61F 13/533, A61F 13/472, A61F 13/475, A61F 13/539, A61F 13/47, A61F 13/511

(54) **ABSORBENT ARTICLE**
ABSORBIERENDER ARTIKEL
ARTICLE ABSORBANT

(30) Priority: 30.03.2017 JP 2017066736
(43) Date of publication of application: 05.02.2020
(73) Proprietor: Daio Paper Corporation, Ehime 799-0492 (JP)
(72) Inventor: WATANABE, Ikuya, Sakura-shi Tochigi 329-1411 (JP)
(74) Representative: Epping - Hermann - Fischer
(86) International application number: PCT/JP2018/010487
(87) International publication number: WO 2018/180602

(56) References cited:
- JP-A- 2009 000 351
- JP-A- 2013 075 009
- JP-A- 2014 198 065
- JP-A- 2015 097 716
- JP-A- 2015 123 163
- US-A1- 2016 143 790
- US-B2- 7 547 815

## Description

### TECHNICAL FIELD

The present invention mainly relates to absorbent articles used for, for example, a sanitary napkin, a panty liner, an incontinence pad, and toiletries.

### BACKGROUND ART

A known absorbent article includes an impermeable back-side sheet such as a polyethylene sheet or a polyethylene-sheet-laminated nonwoven fabric, a permeable upper-side sheet such as a nonwoven fabric or a permeable plastic sheet, and an absorber made of, for example, cotton pulp and disposed between the impermeable back-side sheet and the permeable upper-side sheet.

For a person who discharges a large amount of blood and a person who cannot leave the workplace for a long time due to the type of work, a night sanitary napkin is an essential item. This type of sanitary napkin needs to have a water absorption capacity higher than a normal product and a capability to retain an absorbed body fluid without leakage.

Various improvements have been made on such absorbent articles, and technologies for forming various types of compressed grooves by indenting the absorbent article from the skin-contacting side toward the non-skin side have been proposed to prevent leakage of body fluids and enable the absorbent article to smoothly deform along the body shape when the absorbent article is worn.

For example, Patent Document 1 discloses an absorbent article including an absorbing layer. The absorbing layer includes a separating part having grooves on a skin-contacting side and small absorbing parts that are partitioned by the separating part and have a basis weight that is greater than the basis weight of the separating part. The grooves extend continuously in the longitudinal direction of the absorbent article and in a width direction orthogonal to the longitudinal direction. The small absorbing parts are shaped like blocks each of which is surrounded by the grooves and are arranged to form a grid.

Also, Patent Document 2 discloses an absorbent article that includes an annular first compressed groove and an annular second compressed groove disposed inside of the first compressed groove.

### [RELATED-ART DOCUMENTS]

### [Patent Documents]

[Patent Document 1] Japanese Laid-Open Patent Publication No. 2014-097241
[Patent Document 2] Japanese Laid-Open Patent Publication No. 2012-055565

US 2016/0143790 A1 relates to absorbent products, such as an incontinence pad, a sanitary napkin, a panty liner and a disposable diaper for absorbing urine, menstrual blood, vaginal discharge and the like, that include a concave groove-shaped body fluid inflow portion for receiving a large amount of body fluid with an absorber. JP 2014 198065 A relates to an absorbent article

JP 2013 075009 describes an absorbent article with spaced apart front, rear and rear-end surface embossments, in addition to a pair of absorber embossments adjacent to the rear surface embossments.

### DISCLOSURE OF INVENTION

### PROBLEMS TO BE SOLVED BY THE INVENTION

However, with the absorbent article disclosed in Patent Document 1, because the grooves formed in the absorbing layer extend continuously in the longitudinal direction and the width direction, the body fluid tends to diffuse along the grooves and may leak from the ends of the absorbent article. Also, once a passage of the body fluid is formed in the absorber where the grooves are formed continuously in the longitudinal direction and the width direction, the body fluid tends to diffuse along the passage. This prevents the body fluid from diffusing across the entire absorber and as a result, the water absorption capacity of the absorber is reduced.

Also, with the absorbent article disclosed in Patent Document 2, because outer and inner double compressed grooves are formed by compressing the absorbent article from the upper surface of the upper-side sheet, wearability of the absorbent article is reduced. In a normal sanitary napkin, a higher middle portion, where the thickness of the absorber is increased, is formed to improve the fit of the sanitary napkin to the blood discharge opening, and an annular embossment surrounding the higher middle portion is provided to maintain the shape of the higher middle portion. With this configuration, the middle portion of the absorber rises toward the skin to fit the blood discharge opening of a wearer along flexible axes formed by embossment lines that constitute the sides of the annular embossment and extend in the longitudinal direction, and the side portions of the absorbent article deform toward the non-skin side to fit the inside of the groin. However, in the absorbent article disclosed in Patent Document 2, an annular compressed groove is also formed outside of the annular compressed groove surrounding the higher middle portion. This outer compressed groove is present in the side portions deformed along the flexible axes formed by the inner compressed groove. Therefore, the outer compressed groove tends to contact and rub the inside of the groin and reduce wearability.

Accordingly, a primary object of the present invention is to provide a highly-wearable absorbent article that is configured to enable a body fluid to diffuse across the entire absorber and thereby improve the water absorption capacity, and to prevent leakage of the body fluid.

### MEANS FOR SOLVING THE PROBLEMS

To solve the above problems, claim 1 of the present invention provides an absorbent article that includes an absorber disposed between a permeable upper-side sheet and an impermeable back-side sheet; a surface embossment that is provided at least on each side of an area corresponding to a body fluid discharge part, extends in a longitudinal direction of the absorbent article, and is formed by indenting the permeable upper-side sheet and the absorber together; and an absorber embossment that is disposed away from the surface embossment and closer to a center in a width direction than the surface embossment, extends along the surface embossment, and is formed by indenting the absorber such that a space is formed between the permeable upper-side sheet and the absorber. The surface embossment includes a front-end surface embossment, a front surface embossment, a rear surface embossment, and a rear-end surface embossment that are arranged apart from each other in the longitudinal direction of the absorbent article. The absorber embossment includes a front absorber embossment, a rear absorber embossment, and a rear-end absorber embossment that are arranged apart from each other in the longitudinal direction of the absorbent article. One or both front ends and rear ends of the front surface embossment and the front absorber embossment are located in substantially the same positions in the longitudinal direction of the absorbent article. One or both of front ends and rear ends of the rear surface embossment and the rear absorber embossment are located in substantially the same positions in the longitudinal direction of the absorbent article

### ADVANTAGEOUS EFFECT OF THE INVENTION

As described above, the present invention makes it possible to enable a body fluid to diffuse across the entire absorber and thereby improve the water absorption capacity, to prevent leakage of the body fluid, and improve wearability of an absorbent article.

### BRIEF DESCRIPTION OF THE DRAWINGS

FIG. 1 is a partially-cutaway development view of a sanitary napkin 1 according to the present invention;
FIG. 2 is a cross-sectional view taken along line II-II of FIG. 1;
FIG. 3 is a cross-sectional view taken along line III-III of FIG. 1;
FIG. 4 is a cross-sectional view taken along line II-II of FIG. 1 and indicating a state where the sanitary napkin 1 is worn;
FIG. 5 is a plan view of an absorber 4;
FIG. 6 is a plan view of an absorber 4 according to a variation; and
FIG. 7 is a plan view of an absorber 4 according to a variation.

### DESCRIPTION OF EMBODIMENTS

Embodiments of the present invention are described below with reference to the accompanying drawings. In the descriptions below, a sanitary napkin is used as an example of an absorbent article.

### <BASIC STRUCTURE OF SANITARY NAPKIN 1>

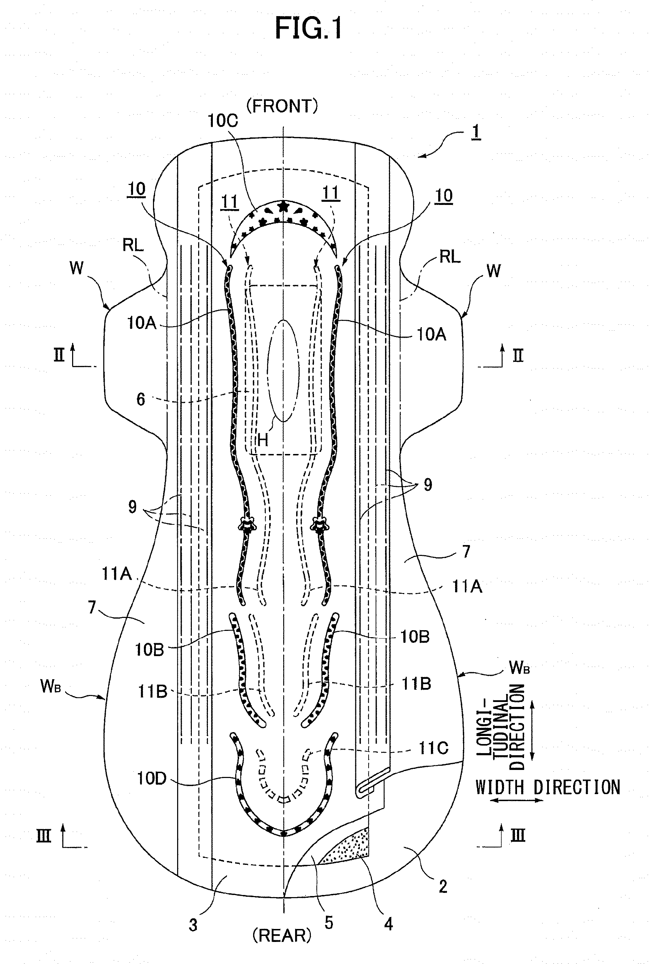

As illustrated in FIGs. 1 through 3, a sanitary napkin 1 according to the present invention includes an impermeable back-side sheet 2 such as a polyethylene sheet, a permeable upper-side sheet 3 that allows, for example, menstrual blood and a vaginal discharge (which may be collectively referred to as a body fluid) to quickly pass therethrough, an absorber 4 disposed between the impermeable back-side sheet 2 and the permeable upper-side sheet 3 and is formed of, for example, cotton pulp or synthetic pulp, and side nonwoven fabrics 7 that are provided on the sides of the skin-contacting surface and extend along almost the entire length of the sanitary napkin 1. At the front and rear edges of the absorber 4, the outer edges of the impermeable back-side sheet 2 and the permeable upper-side sheet 3 are bonded to each other by using, for example, an adhesive such as a hot melt or a bonding technique such as heat sealing or ultrasonic sealing; and at the side edges of the absorber 4, the side edges of the impermeable back-side sheet 2 and the side nonwoven fabrics 7 extending outward from the sides of the absorber 4 are bonded to each other by using, for example, an adhesive such as a hot melt or a bonding technique such as heat sealing or ultrasonic sealing to form flaps where no absorber is present. In the illustrated example, the absorber 4 is enveloped by an enveloping sheet 5, which is formed of, for example, crepe paper or a nonwoven fabric, to maintain the shape and improve the diffusibility of the absorber 4. However, the enveloping sheet 5 may be omitted. Also, although not illustrated in the figures, a second sheet having substantially the same shape as the permeable upper-side sheet 3 and formed of, for example, a hydrophilic nonwoven fabric may be provided next to the non-skin side of the permeable upper-side sheet 3.

The structure of the sanitary napkin 1 is described in more detail below. The impermeable back-side sheet 2 is formed of a sheet material such as polyethylene having at least a water shielding property. A sheet material also having moisture permeability is preferably used. Preferably, the impermeable and moisture-permeable sheet material is a microporous sheet that is obtained by forming a sheet by melt-kneading a mixture of an inorganic filler and an olefinic resin such as polyethylene or polypropylene, and stretching the sheet in a uniaxial or biaxial direction. One or more strips of adhesive layers (not shown) are formed along the longitudinal direction of the napkin on the non-skin side (outer surface) of the impermeable back-side sheet 2 so that the sanitary napkin 1 can be attached to the underwear when wearing the sanitary napkin 1. The impermeable back-side sheet 2 may also be implemented by a polyethylene-sheet-laminated nonwoven fabric formed by stacking a plastic film and a nonwoven fabric.

The permeable upper-side sheet 3 is preferably formed of, for example, a porous or non-porous nonwoven fabric or a porous plastic sheet. Examples of fiber materials of the nonwoven fabric include synthetic fibers made of olefin such as polyethylene or polypropylene, polyester, or polyamide; regenerated fibers such as rayon and cupra, and natural fibers such as cotton. The nonwoven fabric may be produced by any appropriate production method such as spunlacing, spunbonding, thermal bonding, melt blowing, or needle punching. Among these production methods, spunlacing is preferable in terms of flexibility and draping characteristics, and thermal bonding is preferable to produce a bulky nonwoven fabric with a high compression recovery rate. When a large number of permeable holes are formed in the permeable upper-side sheet 3, the permeable upper-side sheet 3 can quickly absorb the body fluid, and the dry touch property of the permeable upper-side sheet 3 is improved. Although the nonwoven fabric may be formed using either long fibers or short fibers, short fibers are preferably used to give a towel-like texture to the nonwoven fabric. Also, to facilitate embossment, olefin fibers, which are formed of, for example, polyethylene or polypropylene having a relatively low melting point, are preferably used. Also, bicomponent fibers may be preferably used. Examples of bicomponent fibers include a core-in-sheath fiber including a high-melting-point fiber as a core and a low-melting-point fiber as a sheath, a side-by-side fiber, and a split fiber.

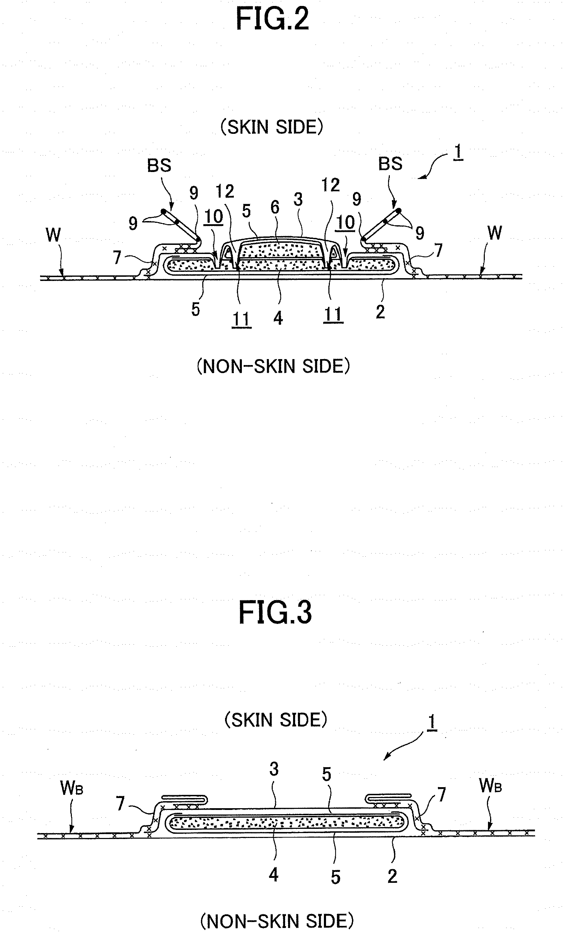

In the examples illustrated in cross-sectional views of FIGs. 2 and 3, the width of the permeable upper-side sheet 3 is slightly longer than the width of the absorber 4 to barely cover the absorber 4. At positions further outside in the width direction, the side nonwoven fabrics 7, which are different from the permeable upper-side sheet 3, are provided to overlap the permeable upper-side sheet 3. The side nonwoven fabrics 7 are formed using a nonwoven fabric material on which water-repellent treatment or hydrophilic treatment is performed depending on a purpose such as preventing penetration of, for example, menstrual blood or a vaginal discharge or improving the feel of the side nonwoven fabrics 7. The side nonwoven fabrics 7 may be formed by any appropriate method using, for example, natural fibers, synthetic fibers, or regenerated fibers as a material. However, to prevent a rough feel and stuffiness, it is preferable to use a nonwoven fabric having a reduced basis weight and air permeability. Specifically, the side nonwoven fabrics 7 are preferably formed using a water-repellent nonwoven fabric that has a basis weight between 13 and 23 g/m² and coated with a silicon-based, paraffin-based, or alkyl chromic chloride-based water repellent to reliably prevent the permeation of a body fluid.

As illustrated in FIGs. 2 and 3, an outer portion relative to the center in the width direction of each side nonwoven fabric 7 is bonded with an adhesive such as a hot melt to an area that extends from an inner position to the outer edge of the impermeable back-side sheet 2. The laminated-sheet portions, where the side nonwoven fabrics 7 and the impermeable back-side sheet 2 overlap each other, form flaps where the absorber 4 is not present on the sides of the absorber 4. The flaps include right and left wing flaps W that are present in a range substantially corresponding to the entire length of an area corresponding to a body fluid discharge part H, and hip-holding flaps WB that are located in positions closer to the hip side (rear side). Adhesive layers (not shown) may be provided on the outer surfaces of the wing flaps W and the hip-holding flaps WB. When the sanitary napkin 1 is attached to shorts, the wing flaps W are folded back along folding lines RL at the bottom to wrap the wing flaps W around the crotch of the shorts, and the hip-holding flaps WB are fixed to the inner side of the shorts.

Meanwhile, the inner portion in the width direction of the side nonwoven fabric 7 is folded and substantially doubled to form a double-sheet portion. One or more, three in this example, elastic strings 9 are disposed in the middle of the double-sheet portion in the height direction. The elastic strings 9 are fixed at the ends or at any appropriate positions in the longitudinal direction. As illustrated in FIG. 3, the front and rear ends of the double sheet portion are bonded to the absorber 4 in a state where the side nonwoven fabric 7 is folded once outward. With this configuration, as illustrated in FIG. 2, the double-sheet portions form a pair of right and left linear three-dimensional gathers BS that are inclined outward and rise in the upward direction.

### <ABSORBER>

The absorber 4 disposed between the impermeable back-side sheet 2 and the permeable upper-side sheet 3 includes, for example, cotton pulp and an absorbent polymer. The absorbent polymer is mixed in the pulp constituting the absorber in the form of, for example, granular powder. The pulp may be made of cellulose fibers such as chemical pulp and dissolving pulp made from wood, or synthetic cellulose fibers such as rayon and acetate. In terms of the function and the price, softwood pulp with a long fiber length is more preferable than hardwood pulp. The mass per unit area of the absorber 4 is between 250 and 650 g/m₂ and preferably between 300 and 400 g/m².

Also, synthetic fibers may be mixed in the absorber 4. The synthetic fibers may be made of, for example, polyolefin such as polyethylene or polypropylene, polyester such as polyethylene terephthalate or polybutylene terephthalate, polyamide such as nylon, or a copolymer of these polymers. Also, a mixture of two types of these synthetic fibers may be used. Further, bicomponent fibers may be used for the absorber 4. Examples of bicomponent fibers include a core-in-sheath fiber including a high-melting-point fiber as a core and a low-melting-point fiber as a sheath, a side-by-side fiber, and a split fiber. When using hydrophobic synthetic fibers, the synthetic fibers are preferably surface-treated with a hydrophilic agent so that the synthetic fibers have an affinity to the body fluid.

A higher middle portion 6 is formed on the skin side of the absorber 4 to increase the thickness of the absorber 4 in a direction toward the skin. The absorber 4 and the higher middle portion 6 may be produced separately and then stacked on each other in an assembly process, or may be formed as a monolithic part by three-dimensionally forming the absorber by fiber stacking. Also, a two-stage fiber-stacked structure, where the higher middle portion 6 is formed on the absorber 4 by fiber stacking, may be employed.

The higher middle portion 6 is disposed next to the skin side of the absorber 4 and located in the middle of the absorber 4 in the width direction. The higher middle portion 6 has a width and a length that are less than those of the absorber 4. If the higher middle portion 6 is too thick, the rigidity of the higher middle portion 6 becomes high, and the contact between the higher middle portion 6 and the body is reduced. If the higher middle portion 6 is too thin, the contact between the higher middle portion 6 and the body fluid discharge part is reduced. Therefore, the thickness of the higher middle portion 6 is preferably between 3 and 25 mm, and more preferably between 5 and 18 mm. Also, the total mass per unit area of the higher middle portion 6 and the absorber 4 in an area where the higher middle portion 6 is located is preferably between 400 and 900 g/m², and more preferably between 600 and 800 g/m².

The higher middle portion 6 is formed in an area including the body fluid discharge part H of the wearer (an area corresponding to the body fluid discharge part H). More specifically, the higher middle portion 6 is formed in an area that is slightly longer than the body fluid discharge part H in the forward and backward directions. The higher middle portion 6 has a shape that is long in the longitudinal direction of the napkin, and the side edges of the higher middle portion 6 are straight lines that extend in the longitudinal direction of the napkin.

The higher middle portion 6 preferably includes at least pulp fibers and synthetic fibers, and the weight ratio of the pulp fibers to the synthetic fibers is preferably 80-20:20-80, and more preferably 40-60:60-40. The higher middle portion 6 may also include an absorbent polymer. Examples of the absorbent polymer include crosslinked polyacrylate, self-crosslinked polyacrylate, saponified product of crosslinked acrylate-vinyl acetate copolymer, crosslinked isobutylene-maleic anhydride copolymer, crosslinked polysulfonate, and partially-crosslinked water-swellable polymer such as polyethylene oxide or polyacrylamide. Among them, polymers including acrylic acid or acrylate are preferable in terms of the water absorption capacity and the water absorption rate. The water absorbing power and the water absorption rate of the absorbent polymer can be adjusted by adjusting the crosslink density and the crosslink density gradient during the production process. If the amount of the absorbent polymer is high, a so-called gel blocking phenomenon may occur. Accordingly, to facilitate penetration of a body fluid from the higher middle portion 6 to the absorber 4, the weight percentage of the absorbent polymer relative to the total weight of the pulp fibers and the synthetic fibers is preferably 1-10%. Mixing the absorbent polymer at a percentage greater than 50% is not preferable because the entanglement among the pulp fibers is reduced, the sheet strength is reduced, and fractures and cracks are generated.

Although not illustrated in the figures, the higher middle portion 6 may have an elongated shape that covers an area including the body fluid discharge part H and an area including the gluteal furrow of the wearer.

### <EMBOSSMENT>

The sanitary napkin 1 includes surface embossments 10 that are provided at least on the sides of an area corresponding to the body fluid discharge part H, extend in the longitudinal direction of the sanitary napkin 1, and are formed by indenting the permeable upper-side sheet 3 and the absorber 4 together; and absorber embossments 11 that are disposed away from the surface embossments 10 and closer to the center in the width direction than the surface embossments 10, extend along the surface embossments 10, and are formed by indenting the absorber 4 such that spaces 12 are formed between the permeable upper-side sheet 3 and the absorber 4.

Each surface embossment 10 is a recessed groove that is formed by indenting the permeable upper-side sheet 3 and the absorber 4 together by applying a pressure from the skin-contacting side (upper surface) of the permeable upper-side sheet 3.

Each absorber embossment 11 is a recessed groove that is formed by indenting only the absorber 4 or both of the permeable upper-side sheet 3 and the absorber 4 by applying a pressure from the skin-contacting side (upper surface) of the absorber 4 or the absorber 4 enveloped by the enveloping sheet 5. As illustrated in FIG. 2, with the permeable upper-side sheet 3 disposed on the skin side of the absorber 4, the spaces 12 are formed between the permeable upper-side sheet 3 and the absorber 4.

Forming the surface embossments 10 and the absorber embossments 11 in the sanitary napkin 1 provides advantageous effects as described below. The body fluid discharged into an area corresponding to the body fluid discharge part H is absorbed by the higher middle portion 6 and the absorber 4, and diffuses in the higher middle portion 6 and the absorber 4. Here, the direction of diffusion of the body fluid diffusing outward in the width direction of the sanitary napkin 1 is changed to the longitudinal direction of the sanitary napkin 1 along the absorber embossments 11 due to the capillarity of compressed fibers in the absorber embossments 11. This enables the body fluid to diffuse across a wide area of the absorber 4, and makes it possible to increase the body fluid absorption capacity. Also, in the area corresponding to the body fluid discharge part H, when the body fluid flowing over the surface of the permeable upper-side sheet 3 without being absorbed by the absorber 4 reaches the surface embossment 10, the body fluid enters the surface embossment 10, the outward flow of the body fluid in the width direction is blocked, and the body fluid diffusing in the longitudinal direction of the sanitary napkin 1 along the surface embossment 10 is absorbed and retained by the absorber 4. Thus, the above configuration enables the body fluid to be absorbed in a wide area of the absorber 4, and thereby makes it possible to prevent leakage of the body fluid.

Also, in the sanitary napkin 1, the middle portion of the sanitary napkin 1 rises toward the skin to fit the body fluid discharge part H along the flexible axes formed by the surface embossments 10, and the side portions of the sanitary napkin 1 deform toward the non-skin side to fit the inside of the groin. Here, because the absorber embossments 11 are formed inside of the surface embossments 10, and no embossment is formed outside of the surface embossments 10, portions of the sanitary napkin 1 outside of the surface embossments 10 softly fit the inside of the groin, and the wearability (feel or wear comfort) of the sanitary napkin 1 is improved. Also, because the surface of the sanitary napkin 1 inside of the surface embossments 10 is substantially flat, the sanitary napkin 1 can tightly contact the body fluid discharge part H, and the wearability is improved.

Further, because the spaces 12 are formed between the permeable upper-side sheet 3 and the absorber 4 as a result of forming the absorber embossments 11 by indenting the absorber 4, the body fluid diffused along the absorber embossments 11 is hardly visible from the upper side and is sufficiently concealed. This gives the wearer a sense of security that leakage is unlikely.

Next, the surface embossments 10 and the absorber embossments 11 are described in more detail.

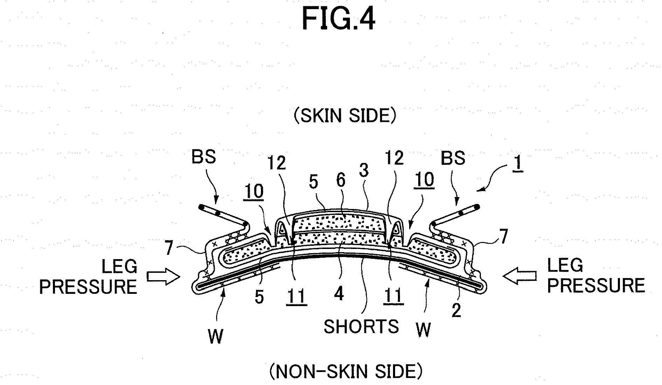

The surface embossments 10 are formed in positions outside of the higher middle portion 6 of the sanitary napkin 1. In the area corresponding to the body fluid discharge part H, the surface embossments 10 (front surface embossments 10A described later) on the sides of the higher middle portion 6 are disposed close to the sides of the higher middle portion 6 and outside of the higher middle portion 6 in the width direction. With this configuration, as illustrated in FIG. 4, when the sanitary napkin 1 is worn, leg pressure is applied to the sides of the sanitary napkin 1. As a result, the middle portion of the sanitary napkin 1, where the higher middle portion 6 is formed, rises toward the skin along the flexible axes implemented by the surface embossments 10, and the side portions of the sanitary napkin 1 deform to fit the inside of the groin.

The surface embossments 10 may be formed at least on the sides of the area corresponding to the body fluid discharge part H. Here, the area corresponding to the body fluid discharge part H is located in the middle of the sanitary napkin 1 in the width direction and includes the entire body fluid discharge part H that contacts a body fluid discharge opening such as the vaginal opening of the wearer when the sanitary napkin 1 is worn. The range of the area corresponding to the body fluid discharge part H in the longitudinal direction of the sanitary napkin 1 substantially corresponds to the range of the wing flaps W in the longitudinal direction when the sanitary napkin 1 includes the wing flaps W on its sides. In the embodiment illustrated in FIG. 1, the surface embossments 10 are formed to extend from the area corresponding to the fluid discharge part H to the area corresponding to the gluteal furrow.

In the sanitary napkin 1 illustrated in FIG. 1, the surface embossments 10 include, on each side of an area extending from the area corresponding to the body fluid discharge part H to the area corresponding to the gluteal furrow, a front surface embossment 10A that is formed by a continuous line extending substantially in the longitudinal direction of the napkin 1; a rear surface embossment 10B that is disposed at a distance behind the front surface embossment 10A and formed on each side of the area corresponding to the gluteal furrow by a continuous line extending substantially in the longitudinal direction of the napkin 1; a front-end surface embossment 10C that is disposed at a distance in front of the front surface embossment 10A and has a planar crescent shape formed substantially in the width direction of the sanitary napkin 1; and a rear-end surface embossment 10D that is disposed at a distance behind the rear surface embossment 10B, crosses the longitudinal center line of the napkin 1 in the width direction, and has a curved planar shape protruding backward.

As illustrated in FIG. 1, the front surface embossment 10A may be provided at least on each side of the higher middle portion 6, and may extend up to a position at which the rear end of the front surface embossment 10A slightly covers the front end of the buttock furrow of the wearer.

The front surface embossment 10A may be shaped like a straight line extending along the longitudinal direction of the sanitary napkin 1. However, the front surface embossment 10A is preferably formed in a shape that includes one or more curved portions protruding inward and outward in the width direction of the sanitary napkin 1 (i.e., portions that curve inward and outward in the width direction) so that the middle portion, which rises when the sanitary napkin 1 is worn, can better fit the shape of the human body.

The rear end portion of the front surface embossment 10A is preferably inclined inward in the width direction. With the rear end portion inclined inward in the width direction, the body fluid diffused along the front surface embossment 10A tends to flow inward in the width direction at the rear end portion. When the body fluid diffuses further backward, this configuration can prevent the body fluid from diffusing outward in the width direction, and can prevent the leakage of the body fluid from the side edge of the absorber 4. Further, the front end portion of the rear surface embossment 10B adjacent to the rear end of the front surface embossment 10A is preferably inclined outward in the width direction at a position outside of the rear end of the front surface embossment 10A in the width direction. This configuration allows the body fluid diffused from the front surface embossment 10A toward the rear surface embossment 10B to flow into an area inside of the rear surface embossment 10B in the width direction, and makes it possible to more reliably prevent the diffusion of the body fluid into an area outside of the rear surface embossment 10B in the width direction.

For similar reasons, the rear end portion of the rear surface embossment 10B and the front end portion of the rear-end surface embossment 10D are preferably arranged such that the rear end portion of the rear surface embossment 10B is inclined inward in the width direction, and the front end portion of the rear-end surface embossment 10D is inclined outward in the width direction at a position outside of the rear end of the rear surface embossment 10B in the width direction.

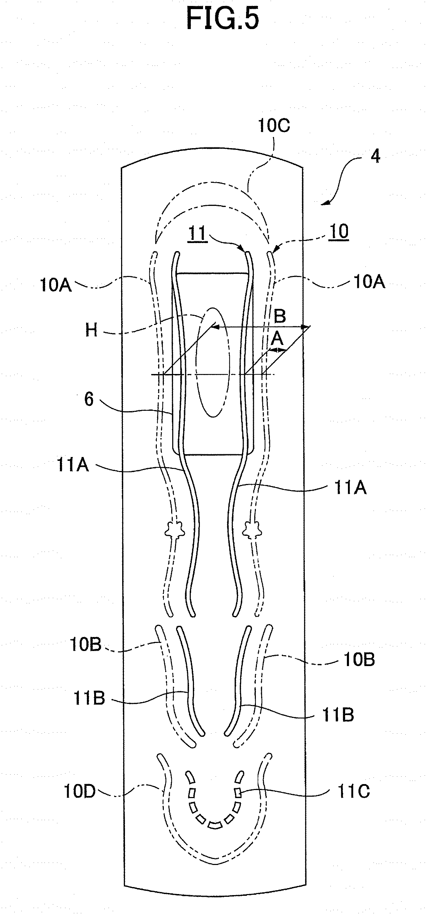

As illustrated in FIG. 5, a distance B between the surface embossments 10 on the sides of the area corresponding to the body fluid discharge part H is preferably set at a value that is substantially the same as the width of a body fluid discharge opening of a human body so that the middle portion between the surface embossments 10 rises along the flexible axes implemented by the surface embossments 10 toward the skin and tightly contacts the body fluid discharge part H when the sanitary napkin 1 is worn Specifically, the distance B is preferably between about 40 mm and about 60 mm.

Next, the absorber embossment 11 is described with reference to FIG. 5. As illustrated in FIG. 2, the absorber embossment 11 is preferably disposed to pass through the higher middle portion 6 and formed by indenting the higher middle portion 6 and the absorber 4 together. The absorber embossments 11 are formed in positions that are closer to the center in the width direction than the surface embossments 10 that are provided to cause the middle portion, where the higher middle portion 6 is formed, to rise toward the skin and fit the skin surface. The absorber embossments 11 are not provided to cause the absorber to deform when the sanitary napkin 1 is worn. Instead, the absorber embossments 11 are provided to cause the body fluid absorbed in the absorber to diffuse in the longitudinal direction of the sanitary napkin 1. Accordingly, the absorber embossments 11 are disposed to pass through the sides of the higher middle portion 6 that closely contacts a body fluid discharge opening such as a vaginal opening of a wearer, and can quickly diffuse the body fluid absorbed by the higher middle portion 6 in the longitudinal direction of the sanitary napkin 1 and effectively prevent the body fluid from diffusing in the width direction and leaking from the side edges.

Similarly to the surface embossment 10, the absorber embossment 11 may be formed at least on each side of the area corresponding to the body fluid discharge part H. In the example illustrated in FIG. 5, the absorber embossment 11 is formed to extend from the area corresponding to the fluid discharge part H to the area corresponding to the gluteal furrow.

In the example illustrated in FIG. 5, the absorber embossments 11 include, on each side of an area extending from the area corresponding to the body fluid discharge part H to the area corresponding to the gluteal furrow, a front absorber embossment 11A that is formed by a continuous line extending substantially in the longitudinal direction of the napkin 1; a rear absorber embossment 11B that is disposed at a distance behind the front absorber embossment 11A and formed on each side of the area corresponding to the gluteal furrow by a continuous line extending substantially in the longitudinal direction of the napkin 1; and a rear-end absorber embossment 11C that is disposed at a distance behind the rear absorber embossment 11B, crosses the longitudinal center line of the napkin 1 in the width direction, and has a curved planar shape protruding backward. In the illustrated example, the front surface embossment 10A and the rear surface embossment 10B are disposed apart from each other and similarly to this arrangement, the front absorber embossment 11A and the rear absorber embossment 11B are disposed apart from each other.

As illustrated in FIG. 5, the front absorber embossment 11A passes through at least the higher middle portion 6, and may extend to a position at which the rear end of the front absorber embossment 11A slightly covers the front end of the gluteal furrow of the wearer.

The absorber embossment 11 has a shape that extends along the surface embossment 10. The absorber embossment 11 is preferably formed in a shape that substantially matches or is similar to the planar shape of the surface embossment 10. When the absorber embossment 11 has a shape that extends along the surface embossment 10, the distance between the surface embossment 10 and the absorber embossment 11 in the width direction of the sanitary napkin 1 becomes substantially constant in the longitudinal direction of the sanitary napkin 1. Making the distance between the surface embossment 10 and the absorber embossment 11 approximately constant makes it possible to prevent the body fluid diffusing along the absorber embossment 11 from diffusing toward the surface embossment 10 in a portion where the distance is short, and thereby makes it possible to prevent leakage of the body fluid due to concentration of the body fluid in this portion.

A distance A in the width direction of the sanitary napkin 1 between the surface embossment 10 and the absorber embossment 11 is preferably between 5 and 15 mm. If the distance A is less than 5 mm, the body fluid diffusing along the absorber embossment 11 in the longitudinal direction of the sanitary napkin 1 tends to move to the surface embossment 10 in an outer position, and leakage may occur when the body fluid diffuses further outward. If the distance A is greater than 15 mm, because the distance (the width of the higher middle portion 6) between the surface embossments 10 disposed on the sides of the area corresponding to the body fluid discharge part H is substantially the same as the width of the body fluid discharge opening of the human body or corresponds to the width of the body fluid discharge opening of the human body, the absorber embossments 11 are positioned close to each other in the middle portion of the sanitary napkin 1. As a result, when the surface of the napkin 1 is brought into contact with the body fluid discharge opening, the wearer tends to feel the hardness of the absorber embossments 11, and the wear comfort of the sanitary napkin 1 on the skin may be reduced.

Preferably, a part the absorber embossment 11 (the front absorber embossment 11A) formed in the area corresponding to the body fluid discharge part H is included in the area corresponding to the body fluid discharge part H, and the length of the absorber embossment 11 is greater than or equal to one half of the length of the surface embossment 10 (the front surface embossment 10A) formed in the area corresponding to the body fluid discharge part H. This configuration makes it possible to reliably cause the body fluid absorbed by the absorber 4 and the higher middle portion 6 in the area corresponding to the body fluid discharge part H to diffuse in the longitudinal direction of the sanitary napkin 1, and thereby prevent leakage of the body fluid from the edges in the width direction.

The surface embossment 10 and the absorber embossment 11 provided along the inside of the surface embossment 10 may be formed such that one or both of the front ends and the rear ends of the surface embossment 10 and the absorber embossment 11 are located substantially in the same position(s) in the longitudinal direction of the sanitary napkin 1, or may be formed such that the absorber embossment 11 is shorter than the surface embossment 10. Forming the surface embossment 10 and the absorber embossment 11 such that one or both of the front ends and the rear ends of the surface embossment 10 and the absorber embossment 11 are located substantially in the same position(s) in the longitudinal direction of the sanitary napkin 1 can improve the fit of the sanitary napkin 1 to the body and cause the body fluid to diffuse across the entire absorber. Here, the description that the ends are located substantially at the same position in the longitudinal direction of the sanitary napkin 1 indicates that the ends are positioned on one virtual line drawn in the width direction. The distance between the ends in the longitudinal direction of the napkin may be preferably less than or equal to 5 mm and more preferably less than or equal to 3 mm. In the example illustrated in FIG. 5, the front surface embossment 10A and the front absorber embossment 11A provided along the inside of the front surface embossment 10A are formed such that the front and rear ends of the front surface embossment 10A and the front and rear ends of the front absorber embossment 11A are located in substantially the same positions in the longitudinal direction of the sanitary napkin 1. Also, the rear surface embossment 10B and the rear absorber embossment 11B provided along the inside of the rear surface embossment 10B are formed such that the front and rear ends of the rear surface embossment 10B and the front and rear ends of the rear absorber embossment 11B are located substantially in the same positions in the longitudinal direction of the sanitary napkin 1. On the other hand, the front and rear ends of the rear-end surface embossment 10D and the front and rear ends of the rear-end absorber embossment 11C provided along the inside of the rear-end surface embossment 10D are not located in the same positions in the longitudinal direction of the sanitary napkin 1, and the rear-end absorber embossment 11C is shorter than the rear-end surface embossment 10D in the longitudinal direction.

As illustrated in FIG. 5, the rear end portion (the rear-end absorber embossment 11C) of the absorber embossment 11 is preferably shaped like a broken line where compressed parts and non-compressed parts are alternately arranged. This configuration of the rear-end absorber embossment 11C makes it possible to prevent the body fluid from diffusing along the groove direction, and thereby makes it possible to prevent the body fluid from concentrating in the rear end portion and leaking from the rear end of the absorber 4. In the example illustrated in FIG. 5, because the rear-end absorber embossment 11C is disposed independently in the rear-end portion of the absorber 4, the entire rear-end absorber embossment 11C is shaped like a broken line.

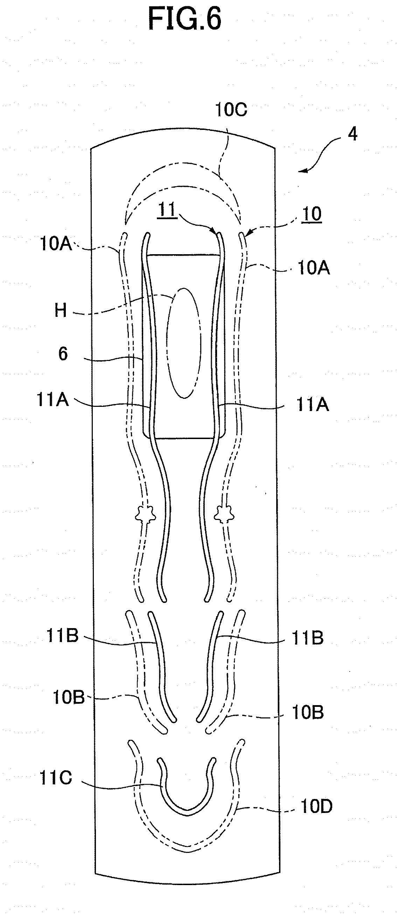

As illustrated in FIG. 6, the rear-end absorber embossment 11C may be shaped like a continuous line.

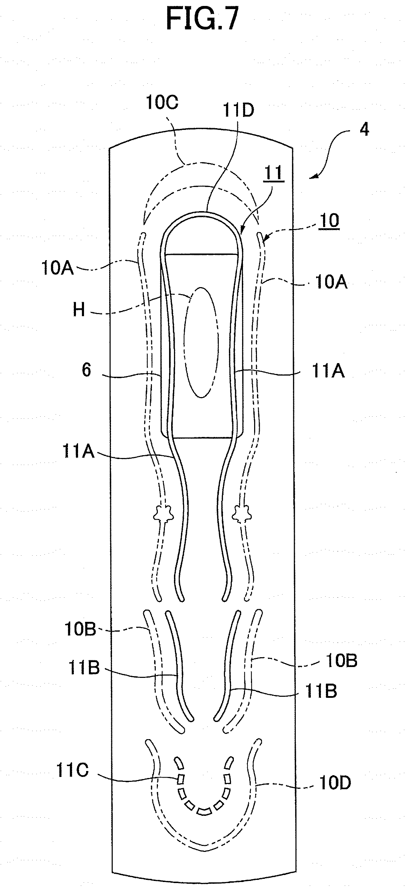

In the above embodiment, as illustrated in FIG. 5, the front ends of the right and left absorber embossments 11 (the front absorber embossments 11A) are disposed in the right and left sides of the longitudinal center line and located apart from each other. However, the front ends of the absorber embossments 11 may be connected to each other as illustrated in FIG. 7. That is, a front-end absorber embossment 11D may be provided to connect the front end of the front absorber embossment 11A on one side to the front end of the front absorber embossment 11A on the other side. The front-end absorber embossment 11D is shaped like a curved line that protrudes toward the front end of the sanitary napkin 1 Connecting the front ends of the right and left absorber embossments 11 facilitates the body fluid absorbed by the absorber 4 in the area corresponding to the body fluid discharge part H to diffuse across the entire absorber 4 along the absorber embossments 11.

The absorber embossment 11 is preferably formed of a continuous line at least in the area corresponding to the body fluid discharge part H, and at least the front absorber embossment 11A is preferably formed of a continuous line. Other portions of the absorber embossment 11 may be formed either of a continuous line or an intermittent broken line.

Each of the surface embossment 10 and the absorber embossment 11 may be a recessed groove that has a flat bottom surface and is formed by applying a substantially uniform pressure over the entire length of the recessed groove, or may be a recessed groove that has an irregular bottom surface and includes high compression parts and low compression parts.

The above-described examples may be used, for example, as a sanitary napkin, a panty liner, an incontinence pad, and toiletries.

### EXPLANATION OF REFERENCE NUMERALS

1 ... sanitary napkin, 2 ... impermeable back-side sheet, 3 ... permeable upper-side sheet, 4 ... absorber, 5 ... enveloping sheet, 6 ... higher middle portion, 7 ... side nonwoven fabric, 9 ... elastic string, 10 ... surface embossment, 11 ... absorber embossment, 12 ... space

## Claims

1. An absorbent article (1), comprising:
an absorber (4) disposed between a permeable upper-side sheet (3) and an impermeable back-side sheet (2);
a surface embossment (10) that is provided at least on each side of an area corresponding to a body fluid discharge part (H), extends in a longitudinal direction of the absorbent article (1), and is formed by indenting the permeable upper-side sheet (3) and the absorber (4) together; and
an absorber embossment (11) that is disposed away from the surface embossment (10) and closer to a center in a width direction than the surface embossment (10), extends along the surface embossment (10), and is formed by indenting the absorber (4) such that a space is formed between the permeable upper-side sheet (3) and the absorber (4), wherein
the surface embossment (10) includes a front-end surface embossment (10C), a front surface embossment (10A), a rear surface embossment (10B), and a rear-end surface embossment (10D) that are arranged apart from each other in the longitudinal direction of the absorbent article (1);
the absorber embossment (11) includes a front absorber embossment (11A), a rear absorber embossment (11B), and a rear-end absorber embossment (11C) that are arranged apart from each other in the longitudinal direction of the absorbent article (1);
one or both front ends and rear ends of the front surface embossment (10A) and the front absorber embossment (11A) are located in substantially the same positions in the longitudinal direction of the absorbent article (1); and
one or both of front ends and rear ends of the rear surface embossment (10B) and the rear absorber embossment (11B) are located in substantially the same positions in the longitudinal direction of the absorbent article (1).

2. The absorbent article (1) as claimed in claim 1, further comprising:
a higher middle portion (6) that is formed in the area corresponding to the body fluid discharge part (H) to increase a thickness of the absorber (4) in a direction toward a skin,
wherein the front absorber embossment (11A) is disposed to pass through the higher middle portion (6) and formed by indenting the higher middle portion (6) and the absorber (4) together.

3. The absorbent article (1) as claimed in claim 1, wherein a distance between the surface embossment (10) and the absorber embossment (11) in the width direction of the absorbent article (1) is between 5 mm and 15 mm.

4. The absorbent article (1) as claimed in claim 1, wherein a portion of the front absorber embossment (11A) is included in the area corresponding to the body fluid discharge part (H), and the front absorber embossment (11A) has a length that is greater than or equal to one half of a length of the front surface embossment (10A).

5. The absorbent article (1) as claimed in claim 1, wherein the rear-end absorber embossment is shaped like a broken line where compressed parts and non-compressed parts are alternately arranged.

6. The absorbent article (1) as claimed in claim 1, wherein front ends of two front absorber embossments (11A) are connected to each other.

## Patentansprüche

1. Absorbierender Artikel (1), umfassend:
einen Absorber (4), der zwischen einer durchlässigen Oberseitenlage (3) und einer undurchlässigen Rückseitenlage (2) angeordnet ist;
eine Oberflächenprägung (10), die zumindest auf jeder Seite eines Bereichs vorgesehen ist, der einem Körperflüssigkeitsabgabeteil (H) entspricht, sich in einer Längsrichtung des absorbierenden Artikels (1) erstreckt und durch gemeinsames Eindrücken der durchlässigen Oberseitenlage (3) und des Absorbers (4) gebildet ist; und
eine Absorberprägung (11), die von der Oberflächenprägung (10) entfernt und näher zu einer Mitte in einer Breitenrichtung als die Oberflächenprägung (10) angeordnet ist, sich entlang der Oberflächenprägung (10) erstreckt und durch Eindrücken des Absorbers (4) gebildet ist, so dass ein Raum zwischen der durchlässigen Oberseitenlage (3) und dem Absorber (4) gebildet ist, wobei die Oberflächenprägung (10) eine vorderendseitige Oberflächenprägung (10C), eine vorderseitige Oberflächenprägung (10A), eine rückseitige Oberflächenprägung (10B) und eine rückendseitige Oberflächenprägung (10D) aufweist, die in Längsrichtung des absorbierenden Artikels (1) voneinander beabstandet angeordnet sind;
die Absorberprägung (11) eine vordere Absorberprägung (11A), eine hintere Absorberprägung (11B) und eine rückendseitige Absorberprägung (11C) aufweist, die in Längsrichtung des absorbierenden Artikels (1) voneinander beabstandet angeordnet sind;
ein oder beide vorderen Enden und hinteren Enden der vorderen Oberflächenprägung (10A) und der vorderen Absorberprägung (11A) in der Längsrichtung des absorbierenden Artikels (1) in im Wesentlichen denselben Positionen angeordnet sind; und
eines oder beide von vorderen Enden und hinteren Enden der hinteren Oberflächenprägung (10B) und der hinteren Absorberprägung (11B) in der Längsrichtung des absorbierenden Artikels (1) in im Wesentlichen denselben Positionen angeordnet sind.

2. Der absorbierende Artikel (1) nach Anspruch 1, ferner umfassend:
einen höheren Mittelabschnitt (6), der in dem Bereich ausgebildet ist, der dem Körperflüssigkeitsabgabeteil (H) entspricht, um eine Dicke des Absorbers (4) in einer Richtung zu einer Haut hin zu erhöhen,
wobei die vordere Absorberprägung (11A) so angeordnet ist, dass sie durch den höheren Mittelabschnitt (6) hindurchgeht und durch gemeinsames Eindrücken des höheren Mittelabschnitts (6) und des Absorbers (4) gebildet wird.

3. Absorbierender Artikel (1) nach Anspruch 1, wobei ein Abstand zwischen der Oberflächenprägung (10) und der Absorberprägung (11) in Breitenrichtung des absorbierenden Artikels (1) zwischen 5 mm und 15 mm beträgt.

4. Absorbierender Artikel (1) nach Anspruch 1, wobei ein Abschnitt der vorderen Absorberprägung (11A) in dem Bereich enthalten ist, der dem Körperflüssigkeitsabgabeteil (H) entspricht, und die vordere Absorberprägung (11A) eine Länge aufweist, die größer als oder gleich einer Hälfte einer Länge der vorderen Oberflächenprägung (10A) ist.

5. Absorptionsartikel (1) nach Anspruch 1, wobei die hintere Absorberprägung wie eine unterbrochene Linie geformt ist, in der komprimierte Teile und nicht komprimierte Teile abwechselnd angeordnet sind.

6. Der absorbierende Artikel (1) nach Anspruch 1, wobei die vorderen Enden von zwei vorderen Absorberprägungen (11A) miteinander verbunden sind.

## Revendications

1. Article absorbant (1), comprenant :
un absorbant (4) disposé entre une feuille supérieure (3) perméable et une feuille verso (2) imperméable ;
un bossage de surface (10) qui est prévu au moins de chaque côté d'une zone correspondant à une partie d'évacuation de fluide corporel (H), s'étend dans une direction longitudinale de l'article absorbant (1), et est formé par indentation conjointe de la feuille supérieure (3) perméable et de l'absorbant (4) ; et
un bossage d'absorbant (11) qui est disposé à l'écart du bossage de surface (10) et plus près d'un centre dans une direction de largeur que le bossage de surface (10), s'étend le long du bossage de surface (3), est formé par indentation de l'absorbant (4) de telle sorte qu'un espace soit formé entre la feuille supérieure (3) perméable et l'absorbant (4), sachant que
le bossage de surface (10) inclut un bossage de surface d'extrémité avant (10C), un bossage de surface avant (10A), un bossage de surface arrière (10B), et un bossage de surface d'extrémité arrière (10D) qui sont agencés séparément les uns des autres dans la direction longitudinal de l'article absorbant (1) ;
le bossage d'absorbant (11) inclut un bossage d'absorbant avant (11A), un bossage d'absorbant arrière (11B), et un bossage d'absorbant d'extrémité arrière (11C) qui sont agencés séparément les uns des autres dans la direction longitudinale de l'article absorbant (1) ;
une ou les deux des extrémités avant et des extrémités arrière du bossage de surface avant (10A) et du bossage d'absorbant avant (11A) sont situées dans sensiblement les mêmes positions dans la direction longitudinale de l'article absorbant (1) ; et
une ou les deux des extrémités avant et des extrémités arrière du bossage de surface arrière (10B) et du bossage d'absorbant arrière (11B) sont situées dans sensiblement les mêmes positions dans la direction longitudinale de l'article absorbant (1).

2. L'article absorbant (1) tel que revendiqué dans la revendication 1, comprenant en outre :
une partie médiane supérieure (6) qui est formée dans la zone correspondant à la partie d'évacuation de fluide corporel (H) pour augmenter une épaisseur de l'absorbant (4) dans une direction vers une peau,
sachant que le bossage d'absorbant avant (11A) est disposé pour passer à travers la partie médiane supérieure (6) et formé par indentation conjointe de la partie médiane supérieure (6) de et l'absorbant (4).

3. L'article absorbant (1) tel que revendiqué dans la revendication 1,
sachant qu'une distance entre le bossage de surface (10) et le bossage d'absorbant (11) dans la direction de largeur de l'article absorbant (1) est comprise entre 5 mm et 15 mm.

4. L'article absorbant (1) tel que revendiqué dans la revendication 1,
sachant qu'une partie du bossage d'absorbant avant (11A) est incluse dans la zone correspondant à la partie d'évacuation de fluide corporel (H), et le bossage d'absorbant avant (11A) a une longueur qui est supérieure ou égale à une moitié d'une longueur du bossage de surface avant (10A).

5. L'article absorbant (1) tel que revendiqué dans la revendication 1,
sachant que le bossage d'absorbant d'extrémité arrière est formé comme une ligne brisée où des parties comprimées et des parties non comprimées sont agencées en alternance.

6. L'article absorbant (1) tel que revendiqué dans la revendication 1,
sachant que des extrémités avant de deux bossages d'absorbant avant (11A) sont raccordées l'une à l'autre.
